(19) Europäisches Patentamt
European Patent Office
Office européen des brevets

(11) Veröffentlichungsnummer: **0 170 228**
**B1**

(12) # EUROPÄISCHE PATENTSCHRIFT

(45) Veröffentlichungstag der Patentschrift: **28.12.88**

(51) Int. Cl.$^4$: **C 07 F 9/38**, C 07 F 9/40, C 07 F 9/58, C 07 F 9/65, A 61 K 31/66

(21) Anmeldenummer: **85109432.6**

(22) Anmeldetag: **26.07.85**

(54) **Neue Diphosphonsäurederivate, Verfahren zu deren Herstellung und diese Verbindungen enthaltende Arzneimittel.**

(30) Priorität: **02.08.84 DE 3428524**

(43) Veröffentlichungstag der Anmeldung: **05.02.86 Patentblatt 86/6**

(45) Bekanntmachung des Hinweises auf die Patenterteilung: **28.12.88 Patentblatt 88/52**

(84) Benannte Vertragsstaaten: **AT BE CH DE FR GB IT LI LU NL SE**

(56) Entgegenhaltungen:
**EP-A- 0 084 822**
**EP-A- 0 085 321**
**DE-A- 1 813 659**

**Die Akte enthält technische Angaben, die nach dem Eingang der Anmeldung eingereicht wurden und die nicht in dieser Patentschrift enthalten sind.**

(73) Patentinhaber: **BOEHRINGER MANNHEIM GMBH, Sandhofer Strasse 116, D-6800 Mannheim 31 (DE)**

(72) Erfinder: **Bosies, Elmar, Dr. rer. nat., Delpstrasse 11, D-6940 Weinheim (DE)**
Erfinder: **Gall, Rudi, Dr. phil., Ulmenstrasse 1, D-6945 Hirschberg 1 (DE)**

**EP 0 170 228 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue Diphosphonsäurederivate, Verfahren zu deren Herstellung sowie Arzneimittel, die diese Substanzen enthalten.

In der DE-OS 3 203 308 bzw. EP-A 85 321 sind Aryl-ethandiphosphonate, z.B. das Thienyl-ethan-diphosphonat mit ausgeprägter antiinflammatorischer Wirkung beschrieben. In der EP-A 0 084 822 sind u.a. Pyrazol-alkyl-diphosphonate mit antiarthritischer Wirkung beschrieben. Daneben zeigen die Verbindungen eine Wirkung auf den Calciumstoffwechsel.

In der DE-PS 1 813 659 sind Diphosphonsäurederivate beschrieben, von denen die 1-Hydroxy-ethan-1,1-diphosphonsäure als Mittel zur Behandlung von Morbus Paget Bedeutung erlangt hat.

Es wurde nun gefunden, dass analoge Derivate dieser Verbindungen, in denen die Alkylkette durch andere aromatische heterocyclische Reste substituiert ist, diese Wirkung bereits in einer niederen Dosis zeigen und somit besser zur breiteren Behandlung von Calciumstoffwechselstörungen geeignet sind. Sie lassen sich vor allem sehr gut dort einsetzen, wo der Knochenauf- und -abbau gestört ist, d.h. sie sind geeignet zur Behandlung von Erkrankungen des Skelettsystems wie z.B. Osteoporose, Morbus Paget, Morbus Bechterew u.a. Aufgrund dieser Eigenschaften finden sie aber auch Verwendung in der Therapie von Knochenmetastasen, der Urolithiasis und zur Verhinderung heterotoper Ossifikationen. Durch ihre Beeinflussung des Calciumstoffwechsels bilden sie weiterhin eine Grundlage für die Behandlung der rheumatoiden Arthritis, der Osteoarthritis und der degenerativen Arthrose.

Gegenstand der vorliegenden Erfindung sind demnach Diphosphonate der allgemeinen Formel I

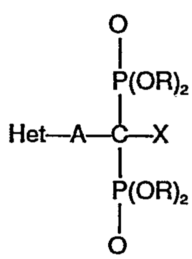

(I),

in der

Het einen Imidazol-, Oxazol-, Isoxazol-, Thiazol-, Pyridin-, 1,2,3-Triazol-, 1,2,4-Triazol- oder Benzimidazol-Rest, der gegebenenfalls durch Alkyl, Alkoxy, Halogen, Hydroxy, Carboxyl, eine gegebenenfalls durch Alkyl- oder Acylgruppen substituierte Aminogruppe oder eine gegebenenfalls durch Alkyl, Nitro, Amino oder Aminoalkyl substituierte Benzylgruppe substituiert sein kann.

A eine geradkettige oder verzweigte, gesättigte oder ungesättigte Kohlenwasserstoffkette mit 2–8 Kohlenstoffatomen,

X Wasserstoff, gegebenenfalls durch Acyl substituiertes Hydroxy, oder gegebenenfalls durch Alkyl- oder Acylgruppen substituiertes Amino und

R Wasserstoff oder Alkyl bedeuten, sowie deren pharmakologisch unbedenkliche Salze.

Alkyl bedeutet in allen Fällen für sich oder bei dem Rest Alkoxy einen Kohlenwasserstoffrest mit 1-4 C-Atomen. Bevorzugt sind die Methyl-, Ethyl- und Isopropylgruppe. Unter einer Acylgruppe versteht man Reste mit 1-6 C-Atomen, insbesondere Formyl, Acetyl, Propionyl, Butyryl und Valeroyl, vorzugsweise jedoch Acetyl und Propionyl.

Unter Halogen sind Fluor, Chlor, Brom und Jod, vorzugsweise Chlor und Brom, zu verstehen.

Die Kette A bedeutet bevorzugt $-(CH_2)_n-$ mit n = 2–5,

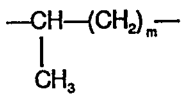 mit m = 2–5,

$—CH{=}CH—CH_2—CH_2—$,

$—CH{=}CH—CH_2—CH_2—CH_2—$,

$—CH{=}CH—CH{=}CH—$,

insbesondere die aufgeführten gesättigten Reste.

Der Substituent X bedeutet vorzugsweise Hydroxy oder Amino, insbesondere Hydroxy.

Verbindungen der allgemeinen Formel I werden nach an sich bekannten Verfahren hergestellt.

Für den Fall, dass X in der allgemeinen Formel I OH bedeutet, stellt man die Substanzen vorzugsweise dadurch her, dass man

a) eine Carbonsäure der allgemeinen Formel II

$Het\text{-}A\text{-}CO_2H$ (II),

in der Het und A die oben angegebenen Bedeutungen haben, mit einem Gemisch aus phosphoriger Säure und einem Phosphorhalogenid umsetzt und anschliessend zur freien Diphosphonsäure verseift, oder

b) ein Carbonsäurechlorid der allgemeinen Formel III

Het-A-COCl (III),

in der Het und A die oben angegebenen Bedeutungen haben, mit einem Trialkylphosphit der allgemeinen Formel IV

$P(OR')_3$ (IV),

in der R′ niederes Alkyl bedeutet, zu einem Acylphosphonat der allgemeinen Formel V

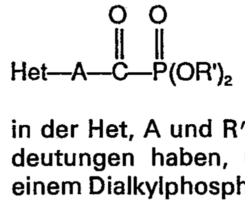

(V)

in der Het, A und R′ die oben angegebenen Bedeutungen haben, umsetzt, anschliessend mit einem Dialkylphosphit der allgemeinen Formel VI

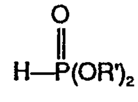

(VI)

in der R′ die oben angegebene Bedeutung hat, zu einem Diphosphonat der allgemeinen Formel VII

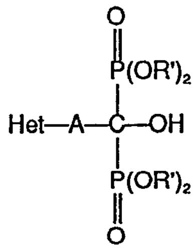

(VII)

in der Het, A und R′ die oben angegebenen Bedeutungen haben, reagieren lässt und gegebenenfalls die entstandenen Tetraester zu Diestern oder Säuren der allgemeinen Formel I verseift, oder für den Fall, dass X in der allgemeinen Formel I gegebenenfalls durch Alkylgruppen substituiertes Amino bedeutet,

c) ein Carbonsäurederivat der allgemeinen Formel VIII

Het—A—Z (VIII)

in der Het und A die oben angegebenen Bedeutungen haben und Z eine Nitril-, Iminoether- oder eine N,N-Dialkylcarboxamidogruppe darstellt, mit einer Phosphorverbindung der allgemeinen Formel IX

$PT_3$ (IX)

in der T = Halogen, OH oder OR′ bedeutet, wobei R′ die oben angegebene Bedeutung hat, umsetzt und gegebenenfalls anschliessend verseift, oder für den Fall, dass X in der allgemeinen Formel I Wasserstoff bedeutet,

d) eine Verbindung der allgemeinen Formel X

Het—A—Y (X)

in der Het und A die oben angegebenen Bedeutungen haben und y einen reaktiven Rest wie z.B. Halogen oder ein Sulfonat darstellt, mit einer Verbindung der allgemeinen Formel XI

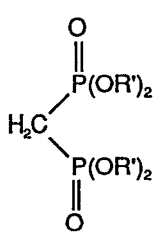

(XI),

in der R′ die oben angegebene Bedeutung hat, zu einem Diphosphonat der allgemeinen Formel XII

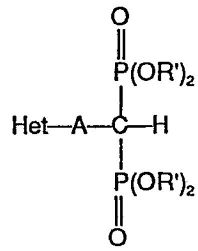

(XII),

in der Het, A und R′ die oben angegebenen Bedeutungen haben, umsetzt und gegebenenfalls die entstandenen Tetraester zu Diestern oder Säuren der allgemeinen Formel I verseift.

Die bei Verfahren a) eingesetzten Carbonsäuren der allgemeinen Formel II werden mit 1-2, vorzugsweise 1,5 Mol phosphoriger Säure und 1-2, vorzugsweise 1,5 Mol Phosphortrihalogenid bei Temperaturen von 80-130°C, vorzugsweise 100-110°C umgesetzt. Man kann die Reaktion auch in Gegenwart von Verdünnungsmitteln wie Halogenkohlenwasserstoffen, insbesondere Chlorbenzol, Tetrachlorethan oder auch Dioxan durchführen. Die anschliessende Hydrolyse erfolgt durch Kochen mit Wasser, zweckmässigerweise jedoch mit halbkonzentrierter Salz- oder Bromwasserstoffsäure.

Phosphorige Säure und Phosphortrihalogenid können in dieser Reaktion durch Phosphorpentoxid, Phosphorpentahalogenid bzw. Phosphoroxihalogenid ersetzt werden.

(DE-A 2 130 794, DE-A 2 658 961, DE-A 2 702 631 und DE-A 2 943 498)

Bei Verfahren b) lässt man das Säurechlorid der allgemeinen Formel II mit dem Trialkylphosphit der allgemeinen Formel IV bei Temperaturen ren zwischen 0 und 60°C, vorzugsweise bei 20-40°C zur Reaktion kommen. Man kann ohne Lösungsmittel oder auch in Gegenwart von inerten Lösungsmitteln wie Diethylether, Tetrahydrofuran, Dioxan oder auch halogenierten Kohlenwasserstoffen, wie z.B. Methylenchlorid arbeiten. Das als Zwischenprodukt entstehende Acylphosphonat der allgemeinen Formel V kann isoliert oder direkt weiter umgesetzt werden. Die anschliessende Reaktion führt man in Gegenwart einer schwachen Base, vorzugsweise einem sec. Amin wie z.b. Dibutylamin bei einer Temperatur von 0 bis 60°C, vorzugsweise bei 10-30°C durch.

Bei Verfahren c) setzt man die Nitrile der allgemeinen Formel VIII mit phosphoriger Säure bei Temperaturen von 110-180°C um. Die Reaktion kann ohne oder in Gegenwart von aprotischen Lösungsmitteln wie z.B. Diglykoldimethylether oder Diglykoldiethylether durchgeführt werden. Man kann die Nitrile jedoch auch mit einem Phosphortrihalogenid, z.B. Phosphortribromid oder Phosphortrichlorid in einem inerten Lösungsmittel wie z.B. Dioxan oder Tetrahydrofuran gegebenenfalls unter Zusatz von Wasser bei Temperaturen von 20-80°C zur Reaktion bringen. Iminoether der allgemeinen Formel VIII lässt man mit Dialkylphosphiten vorzugsweise in Gegenwart äquimolarer Mengen Natrium in inerten Lösungsmitteln wie Diethylether, Dioxan oder auch Benzol reagieren, wobei die Umsetzungen in der Regel bei der Rückflusstemperatur des entsprechenden Lösungsmittels stattfindet. Säureamide der allgemeinen Formel VIII kann man in inerten Lösungsmitteln wie z.B. halogenierten Kohlenwasserstoffen oder Ethern wie z.B. Diethylether mit einem Gemisch aus Phosphorpentahalogenid/phosphoriger Säure oder auch Oxalylchlorid/Trialkylphosphit umsetzen.

Bei Verfahren d) setzt man den Methylendiphosphonsäureester der allgemeinen Formel XI in Form seines Natrium- oder Kaliumsalzes ein. Hierzu wird er mit Natrium, Kalium oder dem entsprechenden Hydrid in einem inerten Lösungsmittel wie z.B. Benzol, Toluol oder Dimethylformamid bei einer Temperatur von 0 bis 40°C vorzugsweise bei 25°C umgesetzt. Das Alkalisalz wird ohne Isolierung mit dem entsprechenden Halogenid bzw. Sulfonat zur Reaktion gebracht. Die Temperatur liegt hierbei bei 20–110°C.

Die bei den Verfahren b), c) und d) gegebenenfalls anfallenden Tetraalkylester können zu Diestern oder den freien Tetrasäuren verseift werden. Die Verseifung zu Diestern geschieht in der Regel dadurch, dass man den Tetraalkylester mit einem Alkalihalogenid, vorzugsweise Natriumjodid in einem geeigneten Lösungsmittel wie z.B. Aceton bei Zimmertemperatur behandelt.

Hierbei entsteht das symmetrische Diester/Dinatriumsalz, das gegebenenfalls durch einen sauren Ionenaustauscher in die Diester/Disäure umgewandelt werden kann. Die Verseifung zu freien Diphosphonsäuren geschieht in der Regel durch Kochen mit Salz- oder Bromwasserstoffsäure. Man kann jedoch auch eine Spaltung mit Trimethylsilylhalogenid, vorzugsweise dem Bromid oder Jodid vornehmen. Die freien Diphosphonsäuren können umgekehrt durch Kochen mit Orthoameisensäurealkylestern wieder in die Tetraalkylester überführt werden. Die freien Diphosphonsäuren der allgemeinen Formel I können als freie Säuren oder in Form ihrer Mono- oder Dialkalisalze isoliert werden. Die Alkalisalze lassen sich in der Regel durch Umfällen aus Wasser/Methanol oder Wasser/Aceton gut reinigen.

Die Verbindungen der allgemeinen Formel I können gegebenenfalls nachträglich ineinander überführt werden. Sie können z.B. alkyliert oder acyliert werden oder, wenn X in der allgemeinen Formel I eine unsubstituierte Aminogruppe bedeutet, durch Diazotieren in die Verbindungen der allgemeinen Formel I mit X = OH umgewandelt werden. Durch hydrogenolytische Abspaltung einer N-Benzylgruppe lassen sich z.B. die entsprechenden unsubstituierten Verbindungen der allgemeinen Formel I darstellen.

Als pharmakologisch verträgliche Salze werden vor allem Alkali- oder Ammoniumsalze verwendet, die man in üblicher Weise z.B. durch Neutralisation der Verbindungen mit anorganischen oder organischen Basen wie z.B. Natrium- oder Kaliumhydrogencarbonat, Natronlauge, Kalilauge, wässrigem Ammoniak oder Aminen wie z.B. Trimethyl- oder Triethylamin herstellt.

Die erfindungsgemässen neuen Substanzen der Formel I und ihre Salze können in flüssiger oder fester Form enteral und parenteral appliziert werden. Hierbei kommen alle üblichen Applikationsformen in Frage, beispielsweise Tabletten, Kapseln, Dragees, Sirupe, Lösungen, Suspensionen etc. Als Injektionsmedium kommt vorzugsweise Wasser zur Anwendung, welches die bei Injektionslösungen üblichen Zusätze wie Stabilisierungsmittel, Lösungsvermittler und Puffer enthält. Derartige Zusätze sind z.B. Tartrat- und Citrat-Puffer, Äthanol, Komplexbildner (wie Äthylendiamintetraessigsäure und deren nichttoxische Salze), hochmolekulare Polymere (wie flüssiges Polyäthylenoxid) zur Viskositätsregelung. Flüssige Trägerstoffe für Injektionslösungen müssen steril sein und werden vorzugsweise in Ampullen abgefüllt. Feste Trägerstoffe sind z.B. Stärke, Lactose, Mannit, Methylcellulose, Talkum, hochdisperse Kieselsäuren, höhermolekulare Fettsäuren (wie Stearinsäure), Gelatine, Agar-Agar, Calciumphosphat, Magnesiumstearat, tierische und pflanzliche Fette, feste hochmolekulare Polymere (wie Polyäthylenglykole); für orale Applikation geeignete Zubereitungen können gewünschtenfalls Geschmacks- und Süssstoffe enthalten.

Die Dosierung kann von verschiedenen Faktoren, wie Applikationsweise, Spezies, Alter und/oder individuellem Zustand abhängen. Die täglich zu verabreichenden Dosen liegen bei etwa 1–1000 mg/Mensch, vorzugsweise bei 10–200 mg/Mensch und können auf einmal oder mehrere Male verteilt eingenommen werden.

Bevorzugt im Sinne der vorliegenden Erfindung sind ausser den in den Beispielen genannten Verbindungen und den durch Kombination aller in den Ansprüchen genannten Bedeutungen der Substituenten ableitbaren Verbindungen die folgenden Diphosphonate:

1-Hydroxy-5-(1H-2-imidazolyl)pentan-1.1-diphosphonsäure
1-Hydroxy-3-(1.3H-5-methyl-imidazolin-2-on-4-yl)propan-1.1-diphosphonsäure
1-Hydroxy-4-(1.3H-5-methyl-imidazolin-2-on-4-yl)butan-1.1-diphosphonsäure
1-Hydroxy-5-(1.3H-5-methyl-imidazolin-2-on-4-yl)pentan-1.1-diphosphonsäure
3-(1.3.5-Trimethyl-imidazolin-2-on-4-yl)-1-hydroxypropan-1.1-diphosphonsäure
4-(1.3.5-Trimethyl-imidazolin-2-on-4-yl)-1-hydroxybutan-1.1-diphosphonsäure
5.(1.3.5-Trimethyl-imidazolin-2-on-4-yl)-1-hydroxypentan-1.1-diphosphonsäure
1-Hydroxy-3-(1.2.4-triazol-3-yl)propan-1.1-diphosphonsäure
1-Hydroxy-5-(1.2.4-triazol-3-yl)pentan-1.1-diphosphonsäure
1-Hydroxy-4-(1.2.4-triazol-1-yl)butan-1.1-diphosphonsäure
1-Hydroxy-4-(1.2.3-triazol-4-yl)butan-1.1-diphosphonsäure
1-Hydroxy-4-(1-methyl-1.2.3-triazol-4-yl)butan-1.1-diphosphonsäure
4-(1-Benzyl-1.2.3-triazol-4-yl)-1-hydroxybutan-1.1-diphosphonsäure
3-(5-Amino-1.2.3-triazol-4-yl)-1-hydroxypropan-1.1-diphosphonsäure
5-(5-Amino-1.2.3-triazol-4-yl)-1-hydroxypentan-1.1-diphosphonsäure
3-(5-Amino-1-methyl-1.2.3-triazol-4-yl)-1-hydroxypropan-1.1-diphosphonsäure
5-(5-Amino-1-methyl-1.2.3-triazol-4-yl)-1-hydroxypentan-1.1-diphosphonsäure
3-(5-Amino-1-benzyl-1.2.3-triazol-4-yl)-1-hydroxypropan-1.1-diphosphonsäure
5-(5-Amino-1-benzyl-1.2.3-triazol-4-yl)-1-hydroxypentan-1.1-diphosphonsäure
1-Hydroxy-3-(1.2.3-triazol-1-yl)propan-1.1-diphosphonsäure
1-Hydroxy-5-(1.2.3-triazol-1-yl)pentan-1.1-diphosphonsäure
3-(2-Amino-4-methyl-5-thiazolyl)-1-hydroxypropan-1.1-diphosphonsäure
4-(2-Amino-4-methyl-5-thiazolyl)-1-hydroxybutan-1.1-diphosphonsäure
5-(2-Amino-4-methyl-5-thiazolyl)-1-hydroxypentan-1.1-diphosphonsäure
1-Hydroxy-3-(2-thiazolyl)propan-1.1-diphosphonsäure
1-Hydroxy-3-(4-thiazolyl)propan-1.1-diphosphonsäure
1-Hydroxy-3-(5-thiazolyl)propan-1.1-diphosphonsäure
3-(2-Acetylamino-4-thiazolyl)-1-hydroxypropan-1.1-diphosphonsäure
3-(2-Amino-4-thiazolyl)-1-hydroxypropan-1.1-diphosphonsäure
4-(2-Amino-4-thiazolyl)-1-hydroxybutan-1.1-diphosphonsäure
5-(2-Amino-4-thiazolyl)-1-hydroxypentan-1.1-diphosphonsäure
5-(1-Methyl-1.2.3-triazol-4-yl)-1-propionyloxypentan-1.1-diphosphonsäure
5-(1-Benzyl-1.2.3-triazol-4-yl)-1-propionyloxypentan-1.1-diphosphonsäure
5-(1-Benzyl-1.2.3-triazol-4-yl)-1-hydroxy-2.4-pentadien-1.1-diphosphonsäure
1-Hydroxy-5-(1.2.3-triazol-2-yl)pentan-1.1-diphosphonsäure
1-Hydroxy-4-(1.2.3-triazol-2-yl)butan-1.1-diphosphonsäure
1-Hydroxy-3-(1-methyl-2-benzimidazolyl)propan-1.1-diphosphonsäure
1-Hydroxy-5-(1-methyl-2-benzimidazolyl)pentan-1.1-diphosphonsäure
3-(5-Benzimidazolyl)-1-hydroxypropan-1.1-diphosphonsäure
5-(5-Benzimidazolyl)-1-hydroxypentan-1.1-diphosphonsäure
1-Hydroxy-3-(2-methyl-5-benzimidazolyl)propan-1.1-diphosphonsäure
1-Hydroxy-5-(2-methyl-5-benzimidazolyl)pentan-1.1-diphosphonsäure
1-Hydroxy-3-(6-methyl-5-benzimidazolyl)propan-1.1-diphosphonsäure
1-Hydroxy-5-(6-methyl-5-benzimidazolyl)pentan-1.1-diphosphonsäure
3-(2.6-Dimethyl-5-benzimidazolyl)-1-hydroxypropan-1.1-diphosphonsäure
5-(2.6-Dimethyl-5-benzimidazolyl)-1-hydroxypentan-1.1-diphosphonsäure
5-(2-Benzimidazoly)-1-hydroxypentan-1.1-diphosphonsäure
3-(1.3H-Benzimidazolin-2-on-5-yl)-1-hydroxypropan-1.1-diphosphonsäure
5-(1.3H-Benzimidazolin-2-on-5-yl)-1-hydroxypentan-1.1-diphosphonsäure
3-(1.3-Dimethylbenzimidazolin-2-on-5-yl)-1-hydroxypropan-1.1-diphosphonsäure
5-(1.3-Dimethyl-benzimidazolin-2-on-5-yl)-1-hydroxypentan-1.1-diphosphonsäure
1-Acetoxy-3-(4-imidazolyl)propan-1.1-diphosphonsäure
1-Amino-3-(4-imidazolyl)propan-1.1-diphosphonsäure
1-Dimethylamino-3-(4-imidazolyl)propan-1.1-diphosphonsäure
1-Acetamido-3-(4-imidazolyl)propan-1.1-diphosphonsäure
3-(4-Imidazolyl)propan-1.1-diphosphonsäure
1-Acetoxy-5-[1-benzyl-4-(1.2.3-triazolyl)]pentan-1.1-diphosphonsäure
1-Amino-5-[1-benzyl-4-(1.2.3-triazolyl)]pentan-1.1-diphosphonsäure
5-[1-Benzyl-4-(1.2.3-triazolyl)]-1-methylaminopentan-1.1-diphosphonsäure
3-(4-Imidazolyl)-1-propionyloxypropan-1.1-diphosphonsäure
1-Hydroxy-3-(2-methyl-4-thiazolyl)propan-1.1-diphosphonsäure
1-Hydroxy-3-(2-methyl-5-thiazolyl)propan-1.1-diphosphonsäure
1-Hydroxy-3-(2-methyl-4-imidazolyl)propan-1.1-diphosphonsäure
1-Hydroxy-4-(4-imidazolyl)butan-1.1-diphosphonsäure
1-Hydroxy-3-(3-methyl-4-isoxazolyl)propan-1.1-diphosphonsäure
3-(3-Chlor-5-isoxazolyl)-1-hydroxypropan-1.1-diphosphonsäure
1-Hydroxy-3-(3-methoxy-5-isoxazolyl)propan-1.1-diphosphonsäure
1-Hydroxy-3-(2-methyl-4-oxazolyl)propan-1.1-diphosphonsäure
3-(4.5-Dimethyl-2-oxazolyl)-1-hydroxypropan-1.1-diphosphonsäure
4-(4.5-Dimethyl-2-oxazolyl)-1-hydroxybutan-1.1-diphosphonsäure
5-(4.5-Dimethyl-2-oxazolyl)-1-hydroxypentan-1.1-diphosphonsäure
3-(2-Benzyl-4-oxazolyl)-1-hydroxypropan-1.1-diphosphonsäure
5-(2-Benzyl-4-oxazolyl)-1-hydroxypentan-1.1-diphosphonsäure

Die nachfolgenden Beispiele zeigen einige der Verfahrensvarianten, die zur Synthese der erfindungsgemässen Verbindungen verwendet werden können. Sie sollen jedoch nicht eine Einschränkung des Erfindungsgegenstandes darstellen. Die Verbindungen fallen in der Regel als hochschmelzende Festprodukte an, deren Struktur durch H- und P-NMR-Spektroskopie gesichert wurde.

Beispiel 1

1-Hydroxy-3-(4-imidazolyl)propan-1.1-diphosphonsäure

3,53 g (20 mMol) 3-(4-Imidazolyl)propionsäure-hyrochlorid werden mit 2,26 g phosphoriger Säure in 10 ml Chlorbenzol unter Rühren auf 110°C erhitzt. Man tropft langsam 4,12 g (30 mMol) Phosphortrichlorid zu und erhitzt weitere 4 h auf 110°C. Nach Abkühlen wird das Chlorbenzol dekantiert und der Rückstand mit 15 ml 6 N Salzsäure 5 h unter Rückfluss gekocht. Man lässt abkühlen, ersetzt mit Aktivkohle, filtriert und engt die Lösung ein. Den Rückstand nimmt man in 10 ml Wasser auf, stellt die Lösung mit einer wässrigen Natriumbicarbonatlösung auf einen pH-Wert von 5,5 ein und ersetzt so lange mit Methanol, bis kein weiterer Niederschlag mehr ausfällt. Der Niederschlag wird abgesaugt, mit Methanol gewaschen und getrocknet. Ausbeute: 3,23 g = 48% d. Theorie. Die Substanz fällt als Mononatriumsalz mit 1 Kristallwasser an.

In analoger Weise erhält man durch Verwendung von

a) 3-(3-Pyridyl)propionsäure
die 1-Hydroxy-3-(3-pyridyl)propan-1.1-diphosphonsäure in einer Ausbeute von 25%. Die Substanz fällt als Mononatriumsalz mit 1,5 Kristallwasser an.

b) 3-[1-Benzyl-4-(1.2.3-triazolyl)]propionsäure (Fp. 110–112°C; hergestellt durch Hydrierung der 3-[1-Benzyl-4-(1.2.3-triazolyl)]acrylsäure)
die 3-[1-Benzyl-4-(1.2.3-triazolyl)]-1-hydroxypropan-1.1-diphosphonsäure in einer Ausbeute von 48%. Die Substanz fällt als Dinatriumsalz mit 1 Kristallwasser an.

c) 5-[1-Benzyl-4-(1.2.3-triazolyl)]valeriansäure (Fp. 83–85°C; hergestellt durch Hydrierung der 5-[1-Benzyl-4-(1.2.3-triazolyl)]-2.4-pentadiensäure)
die 5-[1-Benzyl-4-(1.2.3-triazolyl)]-1-hydroxypentan-1.1-diphosphonsäure in einer Ausbeute von 53%. Die Substanz fällt als Dinatriumsalz mit 1 Kristallwasser an.

d) 3-(4-Pyridyl)propionsäure
die 1-Hydroxy-3-(4-pyridyl)propan-1.1-diphosphonsäure in einer Ausbeute von 56%. Die Substanz fällt als Mononatriumsalz mit 2 Kristallwasser an.

e) 3-(2-Pyridyl)propionsäure
die 1-Hydroxy-3-(2-pyridyl)propan-1,1-diphosphonsäure
in einer Ausbeute von 54%. Die Substanz fällt als Mononatriumsalz mit 2 Kristallwasser an.

f) 3-(2-Benzimidazolyl)propionsäure
die 3-(2-Benzimidazolyl)-1-hydroxypropan-1,1-diphosphonsäure in einer Ausbeute von 24%. Die Substanz fällt als Mononatriumsalz mit 1,5 Kristallwasser an.

1g) 5-(1-Methyl-1,2,3-triazol-4-yl)valeriansäure (Fp: 74–76°C) die 1-Hydroxy-5-(1-methyl-1,2,3-triazol-4-yl)pentan-1,1-diphosphonsäure in einer Ausbeute von 36%. Die Substanz fällt als Dinatriumsalz mit 2 Kristallwasser an.

1h) 3-(1-Methyl-1,2,3-triazol-4-yl)propionsäure
die 1-Hydroxy-3-(1-methyl-1,2,3-triazol-4-yl)propan-1,1-diphosphonsäure in einer Ausbeute von 50%. Die Substanz fällt als Dinatriumsalz mit 2 Kristallwasser an.

1i) 3-(1-Ethyl-1,2,3-triazol-4-yl)propionsäure
die 3-(1-Ethyl-1,2,3-triazol-4-yl)-1-hydroxypropan-1.1-diphosphonsäure in einer Ausbeute von 54%. Die Substanz fällt als Dinatriumsalz mit 1 Kristallwasser an.

1k) 3-[1-(4-Methylbenzyl)-1,2,3-triazol-4-yl]propionsäure die 1-Hydroxy-3-[1-(4-methylbenzyl)-1,2,3-triazol-4-yl]propan-1,1-diphosphonsäure
in einer Ausbeute von 40%. Die Substanz fällt als Dinatriumsalz mit 1,5 Kristallwasser an.

1l) 3-[1-(4-Aminomethyl-benzyl)-1,2,3-triazol-4-yl]propionsäure (Fp: 207–210°C; hergestellt durch katalytische Hydrierung der 3-[1-(4-Cyanbenzyl)-1,2,3-triazol-4-yl]-acrylsäure (Fp: 168–170°C), die durch Oxidation des entsprechenden 3-[1-(4-Cyanbenzyl)-1,2,3-triazol-4-yl]-acroleins (Fp: 152–155°C) erhalten wurde
die 3-[1-(4-Aminomethyl-benzyl)-1,2,3-triazol-4-yl]-1-hydroxypropan-1.1-diphosphonsäure in einer Ausbeute von 41%. Die Substanz fällt als Dinatriumsalz mit 1 Kristallwasser an.

1m) 3-[1-(3-Nitrobenzyl)-1,2,3-triazol-4-yl]propionsäure (Fp: 147–150°; hergestellt durch Reduktion mittels Hydroxylamin-O-sulfonsäure der 3-[1-(3-Nitrobenzyl)-1,2,3-triazol-4-yl]acrylsäure (Fp: 155–158°), die durch Oxidation des entsprechenden 3-[1-(3-Nitrobenzyl)-1,2,3-triazol-4-yl]acroleins (Fp: 136–140°C) erhalten wurde
die 1-Hydroxy-3-[1-(3-nitrobenzyl)-1,2,3-triazol-4-yl]-propan-1,1-diphosphonsäure in einer Ausbeute von 22%. Die Substanz fällt als Dinatriumsalz mit 2 Kristallwasser an.

1n) 3-(1,2,4-Triazol-1-yl)propionsäure
die 1-Hydroxy-3-(1,2,4-triazol-1-yl)propan-1,1-diphosphonsäure in einer Ausbeute von 45%. Die Substanz fällt als Dinatriumsalz mit 1.5 Kristallwasser an.

1o) 3-(1,2,4-Triazol-1-yl)buttersäure
die 1-Hydroxy-3-(1,2,4-triazol-1-yl)butan-1,1-diphosphonsäure in einer Ausbeute von 44%. Die Substanz fällt als Dinatriumsalz mit 1.5 Kristallwasser an.

1p) 5-(1,2,4-Triazol-1-yl)valeriansäure
die 1-Hydroxy-5-(1,2,4-triazol-1-yl)pentan-1,1-diphosphonsäure in einer Ausbeute von 53%. Die Substanz fällt als Dinatriumsalz mit 1.5 Kristallwasser an.

1q) 3-(1-Benzyl-imidazol-2-yl)propionsäure
die 3-(1-Benzyl-imidazol-2-yl)-1-hydroxypropan-1,1-diphosphonsäure in einer Ausbeute von 71%. Die Substanz wird als freie Säure mit einem Fp: 230–232°C (Aufschäumen) isoliert.

Beispiel 2

1-Hydroxy-3-[4-(1.2.3-triazolyl)]propan-1.1-diphosphonsäure

Man löst 1 g 3-[1-Benzyl-4-(1.2.3-triazolyl)]-1-hydroxypropan-1.1-diphosphonsäure-dinatriumsalz (s. Beispiel 1b) in 40 ml Wasser und hydriert bei Zimmertemperatur in Gegenwart von 0,5 g 10proz. Pd auf Kohle. Die Wasserstoffaufnahme ist nach ca. 6 h beendet. Der Katalysator wird abgesaugt, das Filtrat eingeengt, getrocknet und der Rückstand mit Methanol verrieben. Man erhält 0,74 g = 99% d. Theorie. Die Substanz liegt als Mononatriumsalz mit 1 Kristallwasser vor.

In analoger Weise erhält man durch Hydrierung der

a) 5-[1-Benzyl-(1,2,3-triazol-4-yl)]-1-hyderoxypentan-1,1 diphosphonsäure Dinatriumsalz; s. Beispiel 1c) die 1-Hydroxy-5-(1,2,3-triazol-4-yl)-pentan-1,1-diphosphonsäure in einer Ausbeute von 86%. Die Substanz fällt als Dinatriumsalz mit 2 Kristallwasser an.

b) 3-(1-Benzyl-imidazol-2-yl)-1-hydroxypropan-1,1-diphosphonsäure (s. Beispiel 1q) die 1-Hydroxy-3-(imidazol-2-yl)propan-1,1-diphosphonsäure in einer Ausbeute von 26%, Fp: 238°C Sintern, 240–244°C unter Aufschäumen.

c) 1-Hydroxy-3-[1-(3-nitrobenzyl)-1,2,3-triazol-4-yl]-propan-1,1-diphosphonsäure (Dinatriumsalz; s. Beispiel 1m) die 3-[1-(3-Aminobenzyl)-1,2,3-triazol-4-yl]-1-hydroxypropan-1,1-diphosphonsäure in einer Ausbeute von 60%. Die Substanz fällt als Dinatriumsalz mit 2 Kristallwasser an.

**Patentansprüche**

1. Diphosphonate der allgemeinen Formel I

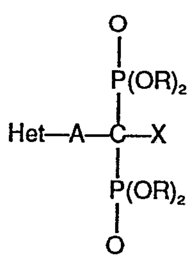

(I),

in der

Het einen Imidazol-, Oxazol-, Isoxazol-, Thiazol-, Pyridin-, 1,2,3-Triazol-, 1,2,4-Triazol- oder Benzimidazol-Rest, der gegebenenfalls durch Alkyl, Alkoxy, Halogen, Hydroxy, Carboxyl, eine gegebenenfalls durch Alkyl- oder Acylgruppen substituierte Aminogruppe oder eine gegebenenfalls durch Alkyl, Nitro, Amino oder Aminoalkyl substituierte Benzylgruppe substituiert sein kann.

A eine geradkettige oder verzweigte, gesättigte oder ungesättigte Kohlenwasserstoffkette mit 2–8 Kohlenstoffatomen,

X Wasserstoff, gegebenenfalls durch Acyl substituiertes Hydroxy, oder gegebenenfalls durch Alkyl- oder Acylgruppen substituiertes Amino und

R Wasserstoff oder Alkyl bedeuten, sowie deren pharmakologisch unbedenkliche Salze.

2. Verfahren zur Herstellung von Diphosphonaten der allgemeinen Formel I

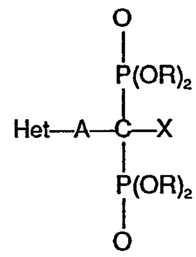

(I),

in der

Het einen Imidazol-, Oxazol-, Isoxazol-, Thiazol-, Pyridin-, 1,2,3-Triazol-, 1,2,4-Triazol- oder Benzimidazol-Rest, der gegebenenfalls durch Alkyl, Alkoxy, Halogen, Hydroxy, Carboxyl, eine gegebenenfalls durch Alkyl- oder Acylgruppen substituierte Aminogruppe oder eine gegebenenfalls durch Alkyl, Nitro, Amino oder Aminoalkyl substituierte Benzylgruppe substituiert sein kann,

A eine geradkettige oder verzweigte, gesättigte oder ungesättigte Kohlenwasserstoffkette mit 2–8 Kohlenstoffatomen,

X Wasserstoff, gegebenenfalls durch Acyl substituiertes Hydroxy, oder gegebenenfalls durch Alkyl- oder Acylgruppen substituiertes Amino und

R Wasserstoff oder Alkyl bedeuten, sowie deren pharmakologisch unbedenkliche Salze, dadurch gekennzeichnet, dass man

a) für den Fall, dass X eine OH-Gruppe bedeutet, eine Carbonsäure der allgemeinen Formel II

$Het\text{-}A\text{-}CO_2H$ (II),

in der Het und A die oben angegebenen Bedeutungen haben, mit einem Gemisch aus phosphoriger Säure und einem Phosphorhalogenid umsetzt und anschliessend zur freien Diphosphonsäure verseift, oder ein Carbonsäurechlorid der allgemeinen Formel III

$Het\text{-}A\text{-}COCl$ (III),

in der Het und A die oben angegebenen Bedeutungen haben, mit einem Trialkylphosphit der allgemeinen Formel IV

$P(OR')_3$ (IV),

in der R′ niederes Alkyl bedeutet, zu einem Acylphosphonat der allgemeinen Formel V

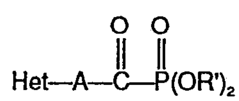

(V)

in der Het, A und R′ die oben angegebenen Bedeutungen haben, umsetzt, anschliessend mit einem Dialkylphosphit der allgemeinen Formel VI

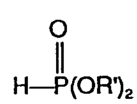

(VI)

in der R′ die oben angegebene Bedeutung hat, zu einem Diphosphonat der allgemeinen Formel VII

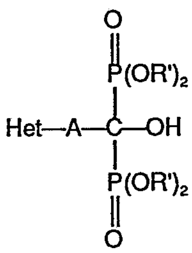

(VII)

in der Het, A und R′ die oben angegebenen Bedeutungen haben, reagieren lässt und gegebenenfalls die entstandenen Tetraester zu Diestern oder Säuren der allgemeinen Formel I verseift, oder

b) für den Fall, dass X gegebenenfalls durch Alkylgruppen substituierte Amino-Gruppe bedeutet, ein Carbonsäurederivat der allgemeinen Formel VIII

Het-A-Z (VIII),

in der Het und A die oben angegebenen Bedeutungen haben und Z eine Nitril-, Iminoether- oder eine N,N-Dialkylcarboxamidogruppe darstellt, mit einer Phosphorverbindung der allgemeinen Formel IX

$PT_3$ (IX),

in der T = Halogen, OH oder OR′ bedeutet, wobei R′ die oben angegebene Bedeutung hat, umsetzt und gegebenenfalls anschliessend verseift, oder

c) für den Fall, dass X Wasserstoff bedeutet, eine Verbindung der allgemeinen Formel X

Het-A-Y (X),

in der Het und A die oben angegebenen Bedeutungen haben und Y einen reaktiven Rest wie z.B. Halogen oder ein Sulfonat darstellt, mit einer Verbindung der allgemeinen Formel XI

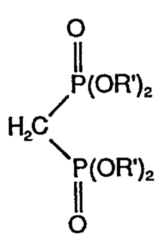

(XI),

in der R′ die oben angegebene Bedeutung hat, zu einem Diphosphonat der allgemeinen Formel XII

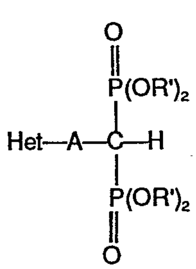

(XII),

in der Het, A und R′ die oben angegebenen Bedeutungen haben, umsetzt, anschliessend gewünschtenfalls die erhaltenen Hydroxy-Verbindungen acyliert, die $NH_2$-Verbindungen alkyliert, acyliert oder diazotiert sowie die erhaltenen Verbindungen der Formel I in pharmakologisch verträgliche Salze überführt.

3. Arzneimittel, enthaltend eine Verbindung gemäss Anspruch 1 und übliche Träger- und Hilfsstoffe.

4. Verwendung von Verbindungen gemäss Anspruch 1 zur Herstellung von Arzneimitteln zur Behandlung von Calciumstoffwechselstörungen.

## Claims

1. Diphosphonates of the general formula I

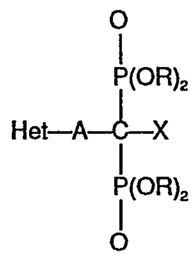

(I),

in which Het signifies an imidazole, oxazole, isoxazole, thiazole, pyridine, 1,2,3-triazole, 1,2,4-triazole or benzimidazole radical, which can optionally be substituted by alkyl, alkoxy, halogen, hydroxyl, carboxyl, an amino group optionally substituted by alkyl or acyl groups or is a benzyl group optionally substituted by alkyl, nitro, amino or aminoalkyl, A a straight-chained or branched, saturated or unsaturated hydrocarbon chain with 2–8 carbon atoms, X hydrogen, hydroxyl optionally substituted by acyl, or amino optionally substituted by alkyl or acyl groups and R hydrogen or alkyl; as well as their pharmacologically acceptable salts.

2. Process for the preparation of diphosphonates of the general formula I

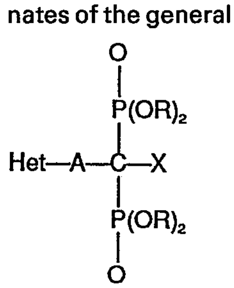

(I),

in which Het signifies an imidazole, oxazole, isoxazole, thiazole, pyridine, 1,2,3-triazole, 1,2,4-triazole or benzimidazole radical, which can optionally be substituted by alkyl, alkoxy, halogen, hydroxyl, carboxyl, an amino group optionally substituted by alkyl or acyl groups or is a benzyl group optionally substituted by alkyl, nitro, amino or aminoalkyl, A a straight-chained or branched, saturated or unsaturated hydrocarbon chain with 2–8 carbon atoms, X hydrogen, hydroxyl optionally substituted by acyl, or amino optionally substituted by alkyl or acyl groups and R hydrogen or

alkyl radical, as well as of their pharmacologically acceptable salts, characterised in that one
a) for the case in which X signifies an OH group, reacts a carboxylic acid of the general formula II

Het-A-$CO_2H$ (II)

in which Het and A have the above-given meaning, with a mixture of phosphorus acid and a phosphorus halide and subsequently saponifies to the free diphosphonic acid; or reacts a carboxylic acid chloride of the general formula III

Het-A-COCl (III)

in which Het and A have the above-given meanings, with a trialkyl phosphite of the general formula IV

$P(OR')_3$ (IV)

in which R′ signifies lower alkyl, to an acyl phosphonate of the general formula V

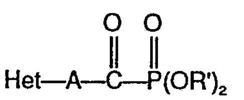
(V)

in which Het, A and R′ have the above-given meanings, subsequently allows to react with a dialkyl phosphite of the general formula VI

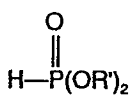
(VI)

in which R′ has the above-given meaning, to a diphosphonate of the general formula VII

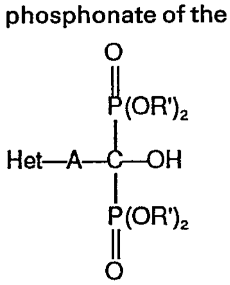
(VII)

in which Het, A and R′ have the above-given meanings, and optionally saponifies the resultant tetraesters to diesters or acids of general formula I; or
b) for the case in which X signifies an amino group optionally substituted by alkyl groups, reacts a carboxylic acid derivative of the general formula VIII

Het-A-Z (VIII)

in which Het and A have the above-given meanings and Z represents a nitrile, imino ether or N,N-dialkylcarboxyamido group, with a phosphorus compound of the general formula IX

$PT_3$ (IX)

in which T signifies halogen, OH or OR′, whereby R′ has the given meaning, and possibly subsequently saponifies, or
c) for the case in which X signifies hydrogen, reacts a compound of the general formula X

Het-A-Y (X)

in which Het and A have the above-given meanings and Y represents a reactive residue, such as e.g. halogen or a sulphonate, with a compound of the general formula XI

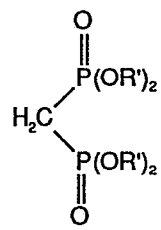
(XI),

in which R′ has the above-given meaning, to a diphosphonate of the general formula XII

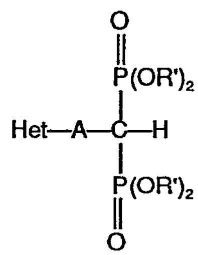
(XII),

in which Het, A and R′ have the above-given meanings, subsequently, if desired, acylates the hydroxy compounds obtained, alkylates, acylates or diazotises the $NH_2$ compounds obtained, as well as converts the compounds obtained into pharmacologically acceptable salts.

3. Medicaments containing a compound according to claim 1 and usual carrier and adjuvant materials.

4. Use of compounds according to claim 1 for the preparation of medicaments for the treatment of calcium metabolism disturbances.

**Revendications**

1. Diphosphonates de formule générale I

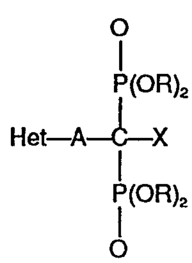
(I),

dans laquelle
Het représente un groupe imidazol, oxazol, isoxazol, thiazol, pyridine, 1,2,3-triazol, 1,2,4-triazol, ou benzimidazol, qui peut être éventuellement substitué par un groupe alkyle, alcoxy, un halogène, un groupe hydroxyle, carboxyle, un groupe amino éventuellement substitué par un groupe alkyle ou acyle, ou un groupe benzyle éventuellement substitué par un groupe alkyle, nitro, amino, ou aminoalkyle,
A représente une chaîne hydrocarbonée linéaire ou ramifiée, saturée ou non, avec 2 à 8 atomes de carbone,
X représente un hydrogène, un groupe hydroxyle éventuellement substitué par un groupe acyle, ou un groupe amino éventuellement substitué par un groupe alkyle ou acyle, et
R représente un hydrogène ou un groupe alkyle, ainsi que leurs sels pharmacologiquement acceptables.

2. Procédé pour la préparation des diphosphonates de formule générale I

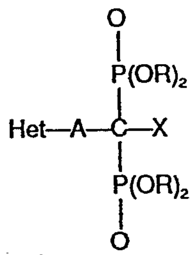

(I),

dans laquelle
Het représente un groupe imidazol, oxazol, isoxasol, thiazol, pyridine, 1,2,3-triazol, 1,2,4-triazol, ou benzimidazol, qui peut être éventuellement substitué par un groupe alkyle, alcoxy, un halogène, un groupe hydroxyle, carboxyle, un groupe amino éventuellement substitué par un groupe alkyle ou acyle, ou un groupe benzyle éventuellement substitué par un groupe alkyle, nitro, amino, ou aminoalkyle,
A représente une chaîne hydrocarbonée linéaire ou ramifiée, saturée ou non, avec 2 à 8 atomes de carbone,
X représente un hydrogène, un groupe hydroxyle éventuellement substitué par un groupe acyle, ou un groupe amino éventuellement substitué par un groupe alkyle ou acyle, et
R représente un hydrogène ou un groupe alkyle, ainsi que leurs sels pharmacologiquement acceptables, caractérisé en ce que

a) dans le cas où X représente un groupe OH, on fait réagir un acide carboxylique de formule générale II

Het-A-$CO_2$H (II),

dans laquelle Het et A ont la signification mentionnée précédemment, avec un mélange d'acide phosphorique et d'un halogénure de phosphore et ensuite, on saponifie en acide diphosphonique libre, ou un chlorure d'acide carboxylique de formule générale III

Het-A-COCl (III),

dans laquelle Het et A ont la signification mentionnée précédemment, avec un phosphite de tialkyle de formule générale IV

$P(OR')_3$ (IV),

dans laquelle R′ représente un groupe alkyle inférieur, en un phosphonate d'acyle de formule générale V

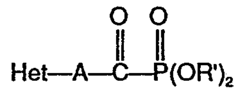

(V)

dans laquelle Het, A et R′ ont la signification mentionnée ci-dessus, que l'on fait ensuite réagir avec un phosphite de dialkyle de formule générale VI

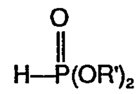

(VI)

dans laquelle R′ a la signification mentionnée ci-dessus, en un diphosphonate de formule générale VII

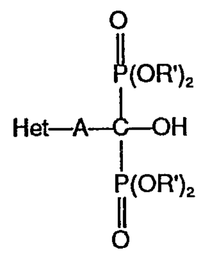

(VII)

dans laquelle Het, A et R′ ont la signification mentionnée ci-dessus, et l'on saponifie éventuellement le tétraester formé en diester ou en acide de formule générale I,
ou

b) dans le cas où X représente, dans la formule générale I, un groupe amino éventuellement substitué par un groupe alkyle, on fait réagir un dérivé d'acide carboxylique de formule générale VIII

Het-A-Z (VIII),

dans laquelle Het et A ont la signification mentionnée ci-dessus, et Z représente un groupe nitrile, iminoéther ou un groupe N,N-dialkylcarboxamido, avec un composé du phosphore de formule générale IX,

$PT_3$ (IX),

dans laquelle T représente un halogène, un groupe OH ou OR′, R′ ayant la signification mentionnée ci-dessus, et ensuite on saponifie éventuellement, ou

c) dans le cas où X représente l'hydrogène, on fait réagir un composé de formule générale X

Het-A-Y (X),

dans laquelle Het et A ont la signification mentionnée ci-dessus, et Y représente un groupe réactif tel que par exemple un groupe halogène ou un groupe sulfonate, avec un composé de formule générale XI

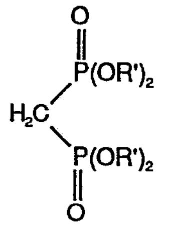

(XI),

dans laquelle R′ a la signification mentionnée cidessus, en un diphosphonate de formule générale XII

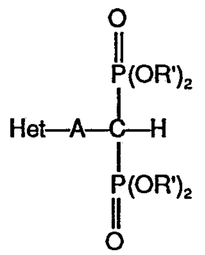

(XII),

dans laquelle Het, A et R′ ont la signification mentionnée ci-dessus, et ensuite, on acyle éventuellement le composé hydroxylé obtenu, on alkyle, acyle ou diazote le composé $NH_2$– obtenu et l'on transforme les composés de formule I en leurs sels pharmacologiquement acceptables.

3. Médicament, comprenant un composé selon la revendication 1, ainsi que les véhicules et matières auxiliaires habituelles.

4. Utilisation des composés selon la revendication 1, pour préparer des médicaments pour le traitement des perturbations du métabolisme du calcium.